(19) Europäisches Patentamt

European Patent Office

Office européen des brevets

(11) **EP 0 824 347 B1**

(12) **EUROPÄISCHE PATENTSCHRIFT**

(45) Veröffentlichungstag und Bekanntmachung des Hinweises auf die Patenterteilung:
**29.10.2003 Patentblatt 2003/44**

(51) Int Cl.[7]: **A61K 31/194**, A61P 27/02, A61P 31/02

(21) Anmeldenummer: **96903985.8**

(22) Anmeldetag: **02.02.1996**

(86) Internationale Anmeldenummer:
**PCT/EP96/00470**

(87) Internationale Veröffentlichungsnummer:
**WO 96/028147 (19.09.1996 Gazette 1996/42)**

(54) **ANTISEPTIKUM FUER DIE AUGENSCHLEIMHAUT ENTHALTEND MAGNESIUMPERPHTHALAT**

ANTISEPTICS FOR THE OPHTALMIC MUCOUS MEMBRANE CONTAINING MAGNESIUM PERPHTHALATE

ANTISEPTIQUE POUR LES MUQUEUSES OPHTALMIQUES, CONTENANT DU PERPHTHALATE DE MAGNESIUM

(84) Benannte Vertragsstaaten:
**AT DE FR GB NL SE**

(30) Priorität: **11.03.1995 DE 19508827**

(43) Veröffentlichungstag der Anmeldung:
**25.02.1998 Patentblatt 1998/09**

(73) Patentinhaber: **Bode Chemie GmbH & Co.**
**22525 Hamburg (DE)**

(72) Erfinder:
• **BRAUEL, Anke**
**D-21077 Hamburg (DE)**
• **PAPE, Wolfgang**
**D-22041 Hamburg (DE)**
• **KRAMER, Axel**
**D-17489 Greifswald (DE)**
• **MEYER, Brigitte**
**D-22303 Hamburg (DE)**
• **PIETSCH, Hanns**
**D-20148 Hamburg (DE)**

(74) Vertreter: **Siewers, Gescha, Dr.**
**Harmsen & Utescher**
**Rechts- und Patentanwälte**
**Alter Wall 55**
**20457 Hamburg (DE)**

(56) Entgegenhaltungen:
**US-A- 5 085 852**

• **DATABASE WPI Week 8831 Derwent Publications Ltd., London, GB; AN 88-215209 XP002004461 & JP,A,63 099 394 (NIPPON PEROXIDE KK) , 30.April 1988**
• **J. APPL. BACTERIOL., Bd. 57, Nr. 3, 1984, Seiten 499-503, XP002004460 M.G.C. BALDRY: "The antimicrobial properties of magnesium monoperoxyphthalate hexahydrate."**

<br/>

# EP 0 824 347 B1

**Beschreibung**

Die Erfindung betrifft Antiseptika für die Augenschleimhaut für den human- und veterinärmedizinischen Einsatzbereich.

Desinfektionsmittel sind Stoffe oder Stoffgemische, die im allgemeinen pantoxisch sind, d.h. sie entfalten ihre Wirkung gegen alle lebenden Zellen. Hautdesinfektionsmittel, die auch als Antiseptika bezeichnet werden, müssen zusätzlich zu den üblichen Anforderungen an Desinfektionsmittel, nämlich breites Wirkungsspektrum, kurze Einwirkungszeiten, geringe Toxizität, Verträglichkeit auch im Umweltbereich usw. noch weitere Anforderungen erfüllen, nämlich eine hervorragende Haut- bzw. Schleimhautverträglichkeit, Wirksamkeit auch in Gegenwart von Eiweißen, keine Eigentoxizität oder Toxizität der Abbauprodukte gegenüber Säugetieren und ein sehr geringes oder völlig fehlendes allergisierendes Potential. Da Antiseptika auf offene Wunden oder Schleimhäute aufgebracht werden, ist nur ein Teil der als Desinfektionsmittel einsetzbaren Verbindungen für diese Zwecke geeignet. Eingesetzt werden beispielsweise die anorganischen Verbindungen Jod und dessen Additions- oder Okklusionsverbindungen oder Quecksilberverbindungen, Kaliumpermanganat, Silbernitrat, Rhodanide und Wasserstoffperoxid und Perchlorsäure, sowie von den organischen Verbindungen vorzugsweise Polyhexanid, quartäre Armoniumverbindungen, Heamidin, Hexetidin, Hydroxychinolin oder Eugenol. Fast alle bisher verwendeten Antiseptika weisen den einen oder den anderen Nachteil auf, entweder sind sie zu wenig wirksam und führen nur zu Wachstumshemmung, nicht aber zur Abtötung der Keime, oder sie haben ein zu hohes allergisierendes Potential, sind auf Wunden oder Schleimhäuten reizend, weisen einen zu intensiven Geruch auf oder sind nicht lagerstabil. Es besteht daher immer noch ein Bedürfnis nach wirksamen Antiseptika mit guter Verträglichkeit, hoher Wirksamkeit und ohne die geschilderten Nebenwirkungen.

Erfindungsgemäß wird jetzt ein Wund- und Schleimhautantiseptikum vorgeschlagen, das gekennzeichnet ist durch einen Gehalt an Magnesiumperphthalat.

Magnesiumperphthalat ist an sich eine bekannte sauerstoffabspaltende Verbindung, die ursprünglich als Bleichmittel auf dem Waschmittelsektor eingesetzt wurde. Später wurde festgestellt, daß Magnesiumperphthalat sich auch als Flächen- und Instrumentendesinfektionsmittel gut eignet und ein breites Wirkungsspektrum besitzt, wobei allerdings das Perphthalat wie fast alle Persäuren in der Regel erst im höheren Temperaturbereich voll wirksam wird und daher entweder bei erhöhten Temperaturen oder unter Zusatz von Aktivatoren zum Einsatz kommt. Es ist auch schon vorgeschlagen worden, Perphthalat allein wie in der PCT-Anmeldung WO87/01562, oder in Kombination mit chlorabspaltenden Verbindungen, wie in der PCT-Anmeldung WO94/00158 beschrieben, zur Desinfektion von Kontaktlinsen einzusetzen.

US-Patent US 5 085 852 beschreibt die Verwendung von Monoperphtalsäuredevivaten wie z.B. Magnesiumperphtalat zur Behandung van mikrobiellen Kraukheiten in der Mundhöhle.

Überraschenderweise ist aber die Eignung von Magnesiumperphthalat als für die Augenschleimhaut Antiseptikum bislang nicht erkannt worden. Gegen einen solchen Einsatz sprach sicherlich die Tatsache, daß das Perphthalat in der Regel erst bei höheren Temperaturen voll wirksam ist und daß aus der Anwendung als Desinfektionsmittel für Kontaktlinsen beispielsweise aus der WO94/00158 bekannt war, daß Wasserstoffperoxid, also das dem Perphthalt zugrunde liegende Wirkprinzip, zu starken Reizungen des Auges bei Verwendung als Desinfektionsmittel führen kann.

Untersuchungen haben jetzt aber ergeben, daß Magnesiumperphthalat selbst in relativ hohen Konzentrationen zwischen 0,2 bis 8% in der Gebrauchslösung völlig reizlos vertragen wird, praktisch kein allergisierendes Potential aufweist und ein breites Spektrum an Keimen wirkungsvoll abtötet.

Die erfindungsgemäßen Antiseptika können als Augenantisepticum und bei Peritonealspülungen eingesetzt werden.

Da Magnesiummonoperperphthalat in kristalliner Form bis zu 3 Jahren haltbar ist, während wäßrige Lösungen bereits nach 8 Std. einen Teil ihrer Aktivität verlieren, werden die erfindungsgemäßen Antiseptika entweder als trokkenes Granulat oder als Tabletten oder Brausetabletten in den Handel gebracht, die in einer bestimmten Wassermenge aufgelöst werden müssen oder sie können, falls erwünscht, auch als Suspension in einem nicht wäßrigen, physiologisch verträglichen und wasserlöslichen Lösungsmittel vorliegen, das dann auf die entsprechende Gebrauchskonzentration zu verdünnen ist. Die Gebrauchskonzentration beträgt in der Regel 0,2 bis 8 Gew.% in im wesentlichen wäßriger Lösung. Als nicht wäßrige, aber wasserlösliche Lösungsmittel für Suspensionen können vorzugsweise solche Verbindungen verwendet werden, die auch im körpereigenen Metabolismus auftreten, wie beispielsweise Glycerin oder Malon- oder Bernsteinsäureester, die ohne weiteres metabolisiert werden und physiologisch unbedenklich sind oder andere untoxische Träger wie Polyethylenglykol oder Polypropylenglykol.

Falls erwünscht und/oder für den geplanten Anwendungszweck notwendig, können die Suspensionen oder die trockenen Pulvermischungen zusätzlich einen gewissen Anteil an nicht ionischen Tensiden enthalten, wenn gleichzeitig die Waschwirkung der fertigen Lösung erhöht werden soll, um beispielsweise Schmutzpartikel von Schürfwunden zu entfernen usw. Der Zusatz an Tensiden sollte in der Regel im Bereich von etwa 2 bis 7 Gew.-% liegen. Geeignete nicht ionische Tenside sind dem Fachmann in großer Zahl bekannt, so daß er ein den jeweiligen Bedürfnissen angepaßtes Tensid auswählen kann. Im übrigen können die flüssigen oder festen Zubereitungen die üblichen Konfektionierungshilfsstoffe enthalten wie Granulier- und Tablettierhilfsmittel, Puffersubstanzen, Suspendierhilfsmittel und Anticakingmittel, Farb- und Aromastoffe etc.

Der pH-Bereich der erfindungsgemäßen Antiseptika sollte im schwachsauren bis schwach-alkalischen liegen, etwas zwischen pH 5,8 bis pH 7,8 und vorzugsweise im Bereich zwischen 6,0 bis 6,3. Falls erforderlich kann dieser pH-Bereich durch Zugabe einer physiologisch verträglichen Säure, wie beispielsweise Zitronensäure eingestellt werden.

Im folgenden wird die Erfindung anhand der Beispiele näher erläutert:

Beispiel 1

Herstellung von Granulaten

Magnesiummonoperphthalat im folgenden abgekürzt als MMPP bezeichnet, kann beispielsweise in Wirbelschichttrockner unter Mitverwendung von Granulierhilfsmitteln granuliert werden. Vorzugsweise wird bei der Herstellung der Granulate ein Wirbelschichttrockner WSG200 der Firma Glatt eingesetzt. Dieses Gerät verfügt über die Möglichkeit einer Luftvortrocknung.

Als Granulierhilfsmittel können Verdickungsmittel wie z.B. Polyvinylpyrrolidon, abgekürzt PVP, und/oder Cellulosederivate wie Methylhydroxyethylcellulose, Propylcellulose oder ähnliche Typen eingesetzt werden. Es ist auch möglich, diese Verdickungsmittel zusammen mit Tensiden zu benutzen wie beispielsweise Cocamidpropylbetain, Alkylethercarbonsäuren, Sulfosuccinate und/oder Alkylpolyglykolether.

Die nachfolgend beschriebenen Mischungen wurden im Wirbelschichttrockner granuliert, wobei die Granuliertemperatur von etwa 30°C auf 50°C während des Vorganges anstieg; für eine MMPP-Menge von 150 kg ist eine Granulierzeit von ca. 2 Stunden notwendig.

Granulate wurden hergestellt aus folgenden Mischungen:

97,0 g MMPP + 3,0 g PVP

97,0 g MMPP + 1,0 g Xanthan

97,0 g MMPP + 1,0 g Xanthan + 2,0 g Ethercarbonsäure

90,0 g MMPP + 1,0 Methylhydroxycellulose + 9,0 Cocamidpropylbetain

Beispiel 2

Herstellung von Tabletten

Die Tablettenherstellung aus MMPP erfolgt in an sich bekannter Weise durch Pressen von Granulaten oder Pulvern in geeigneten technischen Geräten. Als Tablettierhilfsmittel finden die üblichen Binde-, Spreng-, Gleit- und Füllmittel Verwendung. Besonders geeignet sind PVP, substituierte Cellulosen und ggf. Tenside wie beispielsweise Alkylpolyglycolether, Ethercarbonsäuren, Sulfosuccinate und/oder Betaine. Als Sprengmittel können auch Kombinationen aus Säure und Base wie beispielsweise Hydrogencarbonate und Säuren eingesetzt werden.

Die nachfolgenden Rezepturen wurden auf eine Fette EXI Tablettenmschine mit einem Stempel 25 mm, facettiert gepreßt. Die Preßkraft sollte mindestens 22 KN betragen. Die eingesetzte Tablettiermaschine arbeitet mit einer Geschwindigkeit von über > 15 Hub/min.

Hergestellt wurden Tabletten aus folgenden Mischungen:

84,0 g MMPP + 8,0 g Polyethylenglykol + 7,0 g Alkylpolyglykolether +

1,0 g PVP

90,0 g MMPP + 1,0 g Methylhydroxyethylcellulose + 2,0 g PVP

+ 7,0 g Cocamidopropylbetain

25,0 g MMPP + 23,0 g Natriumhydrogencarbonat + 29,0 g Zitronensäure

+ 13,0 g Polyethylenglykol 400

Die angegebenen Substanzen wurden, wie in Beispiel 1 beschrieben, zu Granulaten verarbeitet und dann in an sich bekannter Weise tablettiert.

Beispiel 3

Herstellungen von Suspensionen oder Emulsionen

Zur Herstellung wasserfreier Suspensionen oder Emulsionen werden schleimhautverträgliche Träger eingesetzt wie beispielsweise Paraffine, Silikonöle und/oder Isopropylpalmitat. Als Hilfsstoffe können PVP, substituierte Cellulosen, Siliziumdioxid in feinteiliger Form oder die oben bereits genannten Tenside eingesetzt werden. Die Herstellung der Mischungen erfolgt unter kräftigen Rühren.
Hergestellt wurden folgende Suspensionen:

5,0 g MMPP + 93,0 g Silikonöl + 2,0 g Natriumlaurylsulfat

5,0 g MMPP + 90,0 g Paraffin dünnflüssig + 5,0 g Cocamidopropylbetain

5,0 g MMPP + 7,5 g feinteiliges Siliziumdioxid (Aerosil®)

+ 87,5 g Isopropylpalmitat

5,0 g MMPP + 7,5 g PVP + 87,5 g Isopropylmyristat

Beispiel 4

Toxikologische Untersuchungen

Zur Prüfung der Verträglichkeit von MMPP wurden wäßrige Lösungen mit einem Gehalt an 5 bzw. 8 Gewichtsprozent MMPP auf die primäre Augenreizung in Übereinstimmung mit den empfohlenen Richtlinien der OECD Guideline No. 4 05 Februar 1987 untersucht.
Die Augenreizungen wurden 24,48 und 72 Stunden nach der Applikation ermittelt.
Danach können sowohl 5 als auch 8 %-ige MMPP-Lösungen als nicht augenreizend eingestuft werden.

**Patentansprüche**

1.    Verwendung von Magnesiummonoperphthalat zur Herstellung eines Antiseptikums für die Augenschleimhaut mit einer Konzentration von 0,2 bis 8 Gew.-% in gebrauchsfertiger wäßriger Lösung.

2.    Verwendung nach Anspruch 1, **gekennzeichnet durch** einen weiteren Gehalt an Tensiden.

3.    Verwendung nach Anspruch 1 oder 2, **gekennzeichnet durch** einen Gehalt an 2 bis 7 Gew.-% nicht ionischer Tenside.

4.  Verwendung nach Anspruch 1 bis 3, **gekennzeichnet durch** einen pH-Wert von etwa 5,8 bis 7,8 in gebrauchsfertiger Lösung.

5.  Verwendung von Magnesiummonoperphthalat zur Herstellung eines Antiseptikums für die Augenschleimhaut in im wesentlichen wäßriger Lösung nach Anspruch 1 bis 4, **dadurch gekennzeichnet, daß** es aus einem Konzentrat in Form einer Suspension in nicht wäßrigen, physiologisch verträglichen, wasserlöslichen Lösungsmitteln hergestellt ist.

6.  Verwendung nach Anspruch 5, **dadurch gekennzeichnet, daß** es als Suspension in Glycerin oder Malin- oder Bernsteinsäureestem oder in Polypropylen- oder Polyethylenglykol vorliegt.

**Claims**

1.  Use of magnesium monoperphthalate for the preparation of an antiseptic for the mucous membrane of the eye with a concentration of 0.2 to 8 wt.% in the ready-to-use aqueous solution.

2.  Use according to claim 1, **characterized by** a further content of surfactants.

3.  Use according to claim 1 or 2, **characterized by** a content of 2 to 7 wt.% of nonionic surfactants.

4.  Use according to claim 1 to 3, **characterized by** a pH of about 5.8 to 7.8 in the ready-to-use solution.

5.  Use of magnesium monoperphthalate for the preparation of an antiseptic for the mucous membrane of the eye in substantially aqueous solution according to claim 1 to 4, **characterized in that** it is prepared from a concentrate in the form of a suspension in non-aqueous, physiologically acceptable, water-soluble solvents.

6.  Use according to claim 5, **characterized in that** it is provided as a suspension in glycerol or malic or succinic acid esters or in polypropylene glycol or polyethylene glycol.

**Revendications**

1.  Utilisation de monoperphtalate de magnésium dans la préparation d'un antiseptique pour muqueuse oculaire, en une concentration de 0,2 à 8 % en poids dans une solution aqueuse prête à l'emploi.

2.  Utilisation conforme à la revendication 1, **caractérisée en ce que** la solution contient en outre un tensioactif.

3.  Utilisation conforme à la revendication 1 ou 2, **caractérisée en ce que** la solution contient de 2 à 7 % en poids d'un tensioactif non-ionique.

4.  Utilisation conforme à l'une des revendications 1 à 3, **caractérisée en ce que** le pH de la solution prête à l'emploi vaut à peu près de 5,8 à 7,8.

5.  Utilisation de monoperphtalate de magnésium dans la préparation d'un antiseptique pour muqueuse oculaire en solution essentiellement aqueuse, conforme à l'une des revendications 1 à 4, **caractérisée en ce qu'**on le prépare à partir d'un concentré se présentant sous la forme d'une suspension dans un solvant non-aqueux, physiologiquement compatible et soluble dans l'eau.

6.  Utilisation conforme à la revendication 5, **caractérisée en ce qu'**il s'agit d'une suspension dans du glycérol, dans un ester de l'acide malonique ou de l'acide succinique, ou dans du polypropylèneglycol ou du polyéthylèneglycol.